# EUROPEAN PATENT APPLICATION

(11) **EP 3 285 481 A1**
(43) Date of publication of application: **21.02.2018**
(21) Application number: 16813980.6
(22) Date of filing: 26.01.2016
(51) Int. Cl.: H04N 5/341, A61B 1/04, G02B 23/24, H04N 5/374

(54) **IMAGE CAPTURING DEVICE**

(30) Priority: 22.06.2015 JP 2015124907
(71) Applicant: OLYMPUS CORPORATION, Hachioji-shi Tokyo 192-8507 (JP)
(72) Inventor: SAITO Saeri, Tokyo 192-8507 (JP); TABUCHI Koichiro, Tokyo 192-8507 (JP)
(74) Representative: Gunzelmann, Rainer
(86) International application number: PCT/JP2016/052164
(87) International publication number: WO 2016/208209

(57) **Abstract**

An endoscope 2 connectable to a video processor 3 including a clock A generation circuit 31 and a vertical synchronizing signal A generation portion 32, and includes: a clock C generation circuit 23 configured to generate a clock C set asynchronously with a clock A; a reception circuit 25 configured to receive a vertical synchronizing signal A and also generates a vertical synchronizing signal C that attains gentle synchronization at a frame rate; and a synchronization control signal generation circuit 24 configured to generate a sensor synchronization control signal that controls a CMOS sensor 21 according to the vertical synchronizing signal C.

## Description

### Technical Field

The present invention relates to an image pickup apparatus, in particular to an image pickup apparatus including an image pickup device driven by a clock set independently of and asynchronously with a clock in a connected video processor.

### Background Art

Conventionally, in a medical field and an industrial field, an endoscope including an image pickup device configured to observe a subject has been widely used. In addition, a technology of configuring an endoscope system in which a signal processor called a video processor which performs various kinds of signal processing relating to the endoscope is freely attachably and detachably connected to the endoscope is also known.

In the endoscope system as described above, a conventional endoscope receives supply of a predetermined clock from a connected video processor, and drives a loaded image pickup device by the supplied clock. In this case, synchronization of the endoscope and the video processor is kept at a rate of the clock described above.

On the other hand, video processors of different drive frequencies for each type are present. For example, for the video processor corresponding to an NTSC standard and the video processor corresponding to a PAL standard, the drive frequencies are different from each other.

In the conventional endoscope, even in a case where the endoscope is connected to the video processors of the different drive frequencies, there are no problems in particular even when a drive clock from the video processor is received as it is and used in respective circuits inside the endoscope, a drive circuit of the image pickup device in particular.

That is, in the case of receiving supply of the clock from the video processors of the different drive frequencies such as the NTSC standard and the PAL standard, even though frequencies of the supplied clock are different from each other, such a difference of the frequencies is not a big trouble in the respective circuits loaded on the conventional endoscope.

On the other hand, in recent years, pixels have been increased more and more in the image pickup device loaded on the endoscope, and acceleration and quality improvement are demanded more regarding drive of the image pickup device. That is, for the clock that drives the image pickup device, a performance of higher quality is demanded.

Then, in consideration of the situation, in recent years, an endoscope provided with a high quality clock generation portion independent of a clock generation portion in a video processor is proposed (Japanese Patent Application Laid-Open Publication No. 2014-033788).

As described above, while acceleration is advancing regarding the endoscope, the connected video processors loaded with the clock not satisfying the quality demanded by an endoscope side are also present, and many video processors of the drive frequencies different from each other are still present as described above.

Here, in the case where the quality of the clock supplied by a video processor side is remarkably lower than the clock quality demanded by the endoscope side, when the video processor is just connected without taking any measures, the image pickup device may not be normally driven. Specifically, quality of an image obtained from the image pickup device may be deteriorated, or transmission quality of a video signal may be deteriorated, thereby causing many transmission errors.

In consideration of the point, in the case where, in contrast with a clock generation portion in the video processor, an independent and high-quality clock generation portion is simply provided on the endoscope side and is used for drive of the image pickup device, the demanded quality of the image pickup device can be satisfied.

However, since the clock generated in the clock generation portion generally has a deviation, even if it is assumed that the clock of the same frequency is generated on a specification, the frequencies of the clock on the video processor side and the clock on the endoscope side may be different for the deviation.

That is, even when it is assumed that an operation is performed at a same frame rate, in the case where the quality of the operated clock is greatly different between the endoscope side and the video processor side, when the connection is performed without taking any measures, discrepancy of the frame rate may arise between the image pickup device in the endoscope and an image processing portion in the video processor, and troubles may be caused in image processing or light adjustment of a light source or the like.

In order to cope with a problem of the frame rate discrepancy, it is also conceivable to provide a device such as a PLL circuit on the endoscope side and synchronize the clock supplied from the video processor and the clock generated inside the endoscope by a clock rate.

However, in a present situation, as described above, the video processors of various drive frequencies such as the NTSC standard and the PAL standard are present. Then, since the frequency of an oscillator (VCXO) used in the PLL circuit is demanded to be equal to a clock frequency of the connected video processor, a dedicated endoscope needs to be prepared according to a clock type of the video processor, inviting increase in costs including a management cost.

In addition, a technique of including the oscillators (VCXO) of the respective frequencies in the endoscope in order to cope with the plurality of video processors, and switching the transmitter (VCXO) to be used according to a type of the connected video processor is also conceivable, but a circuit scale becomes huge, and the cost also increases.

The present invention is implemented in consideration of the problem described above, and an object is to provide an image pickup apparatus capable of not causing troubles in image processing or light adjustment of a light source and coping with various video processors of different clock frequencies, in the image pickup apparatus including an image pickup device driven by a clock set independently of and asynchronously with a clock in a connected video processor.

### Disclosure of Invention

### Means for Solving the Problem

An image pickup apparatus in one aspect of the present invention is an image pickup apparatus connectable to a processor including a first clock generation portion configured to generate a predetermined first clock and a first vertical synchronizing signal generation portion configured to generate a predetermined first vertical synchronizing signal, and includes: an image pickup device configured to pick up an image of an object; a second clock generation portion configured to generate a second clock which is a clock that drives the image pickup device and is set independently of and asynchronously with the first clock; a second vertical synchronizing signal generation portion configured to receive the first vertical synchronizing signal and generate a second vertical synchronizing signal including a period synchronized at least at a frame rate with the first vertical synchronizing signal; and a sensor synchronization control signal generation portion configured to generate a sensor synchronization control signal which controls the image pickup device according to the second vertical synchronizing signal.

### Brief Description of the Drawings

Fig. 1 is a block diagram illustrating a schematic configuration of an endoscope system including an image pickup apparatus (endoscope) relating to a first embodiment of the present invention;
Fig. 2 is a timing chart illustrating a relation between a vertical synchronizing signal supplied from a video processor and a vertical synchronizing signal generated on an endoscope side, in the endoscope relating to the first embodiment;
Fig. 3 is a timing chart illustrating a relation between a vertical synchronizing signal inputted to a sensor synchronization control signal generation circuit and a vertical synchronizing signal inside a CMOS sensor in the endoscope relating to the first embodiment;
Fig. 4 is a block diagram illustrating a schematic configuration when a video processor of another drive frequency is connected to the endoscope, in the endoscope system including the image pickup apparatus (endoscope) relating to the first embodiment;
Fig. 5 is a block diagram illustrating a schematic configuration of an endoscope system including an image pickup apparatus (endoscope) relating to a second embodiment of the present invention;
Fig. 6 is a timing chart illustrating a clock and a vertical synchronizing signal supplied to a sensor synchronization control signal generation circuit in the endoscope relating to the second embodiment;
Fig. 7 is a timing chart illustrating a relation between the vertical synchronizing signal inputted to the sensor synchronization control signal generation circuit and the vertical synchronizing signal inside the CMOS sensor in the endoscope relating to the second embodiment;
Fig. 8 is a block diagram illustrating a schematic configuration of an endoscope system including an image pickup apparatus (endoscope) relating to a third embodiment of the present invention; and
Fig. 9 is a block diagram illustrating a schematic configuration of an endoscope system including an image pickup apparatus (endoscope) relating to a fourth embodiment of the present invention.

### Best Mode for Carrying Out the Invention

Hereinafter, embodiments of the present invention will be described.

In addition, the invention is not limited by the embodiments. Further, in description of the drawings, same signs are attached to same parts.

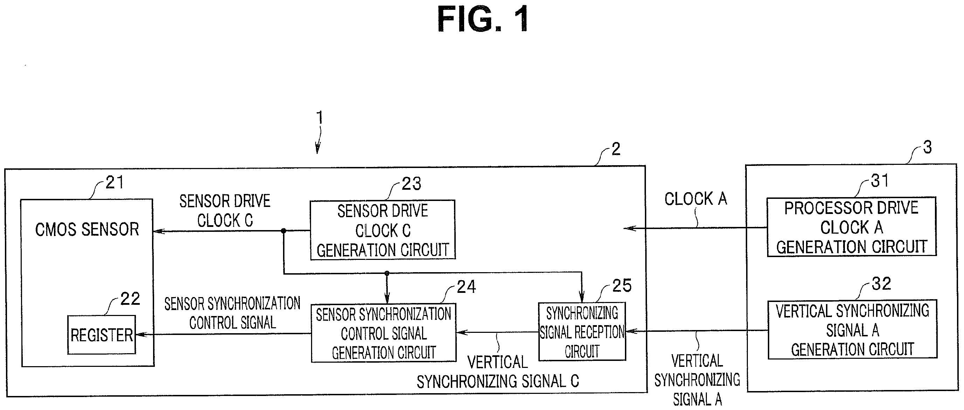

Fig. 1 is a block diagram illustrating a schematic configuration of an endoscope system including an image pickup apparatus of a first embodiment of the present invention.

Note that, the embodiments illustrated below are described with an endoscope including an image pickup device as an example of the image pickup apparatus.

As illustrated in Fig. 1, an endoscope system 1 mainly includes an endoscope 2 configured to pick up an in-vivo image of an object by inserting a distal end portion into a body cavity of a subject and output an image signal of the object image, a video processor 3 configured to execute predetermined image processing on the image signal outputted from the endoscope 2 and generally control an operation of the entire endoscope system 1, and a non-illustrated light source device configured to generate illumination light to be emitted from a distal end of the endoscope 2.

First, the video processor 3 is, in the present embodiment, a video processor corresponding to an NTSC standard for example, including a processor drive clock A generation circuit 31 configured to generate and output a predetermined drive clock A, and a vertical synchronizing signal A generation circuit 32 configured to generate and output a predetermined vertical synchronizing signal A.

In addition, the endoscope 2 includes an image pickup device (CMOS sensor) 21 configured to receive the object image and photoelectrically convert the object image to an electric signal and execute predetermined image processing on the electric signal, a sensor drive clock C generation circuit 23 configured to generate a drive clock (drive clock C) to be supplied to required circuits inside the endoscope 2 in addition to the image pickup device 21, a sensor synchronization control signal generation circuit 24 configured to generate a sensor synchronization control signal to be supplied to the image pickup device 21, and a synchronizing signal reception circuit 25 configured to receive the vertical synchronizing signal A from the video processor 3 and output a vertical synchronizing signal C to be supplied to the sensor synchronization control signal generation circuit 24.

For the image pickup device 21, in the present embodiment, a CMOS (complementary metal oxide semiconductor) image sensor (CIS) is adopted. Hereinafter, the image pickup device 21 is also described as a CMOS sensor 21.

In the present embodiment, the CMOS sensor 21 receives the drive clock C generated in the sensor drive clock C generation circuit 23, and is driven.

In addition, the CMOS sensor 21 includes a register 22 which can be accessed from an outside (in the present embodiment, accessed by the sensor synchronization control signal), and holds setting data for generating a predetermined drive pulse.

The sensor drive clock C generation circuit 23 generates the drive clock C independently of the clock in the video processor 3. The sensor drive clock C generation circuit 23 is configured by a crystal oscillator for example, and outputs to required circuits inside the endoscope 2 including the sensor synchronization control signal generation circuit 24 and the synchronizing signal reception circuit 25 in addition to the CMOS sensor 21.

Note that, in the present embodiment, a center frequency of the drive clock C is set at a frequency higher than the clock A in the video processor 3, and is set asynchronously with the clock A.

Here, as described above, in the present embodiment, the clock A supplied from the video processor 3 and the clock C in the sensor drive clock C generation circuit 23 are asynchronously set, that is, a configuration is such that synchronization is not attained at a clock rate.

Therefore, in a case of such a configuration, for example in the case of counting a time period corresponding to one frame using the clock A of a frequency A in the video processor 3 and counting the time period using the clock C of a frequency C on a side of the endoscope 2, both time periods are slightly different due to a deviation of the mutual clock generation circuits.

Then, when the side of the video processor 3 and the side of the endoscope 2 uniquely continue counting, a difference may become non-negligible. Specifically, drive timing of the CMOS sensor 21 on the side of the endoscope 2 may deviate from vertical synchronizing timing in the video processor 3.

In this case, in the video processor 3 to which an image pickup signal from the CMOS sensor 21 of the endoscope 2 is inputted, a failure may occur when performing image processing relating to the image pickup signal or when executing a light adjustment function of a light source.

Note that the light adjustment function of the light source described above includes not only control of a light emission intensity but also control of changeover timing of a color of illumination light or timing in the case of intermittent light emission. In addition, the control timing of the light source needs to be determined according to drive timing of the CMOS sensor 21.

In consideration of a situation described above, an object of the present invention is to prevent occurrence of the failure described above by surely operating synchronization at a frame rate between the endoscope 2 and the video processor 3.

Hereinafter, a characteristic configuration of the present invention will be described.

Returning to Fig. 1, the sensor synchronization control signal generation circuit 24 is driven by the clock C generated in the sensor drive clock C generation circuit 23, and generates and outputs the sensor synchronization control signal to be supplied to the register 22 in the CMOS sensor 21.

Note that the sensor synchronization control signal generation circuit 24 and the CMOS sensor 21 are connected by communication by a clock synchronous serial communication standard so called I2C (inter-integrated circuit).

In addition, for the CMOS sensor 21, the drive timing is determined based on the vertical synchronizing timing indicated from the sensor synchronization control signal generation circuit 24.

The synchronizing signal reception circuit 25 receives the vertical synchronizing signal A from the video processor 3, is driven by the clock C generated in the sensor drive clock C generation circuit 23, and generates and outputs the vertical synchronizing signal C to be supplied to the sensor synchronization control signal generation circuit 24.

### <Action of First Embodiment>

Next, an action of the first embodiment will be described.

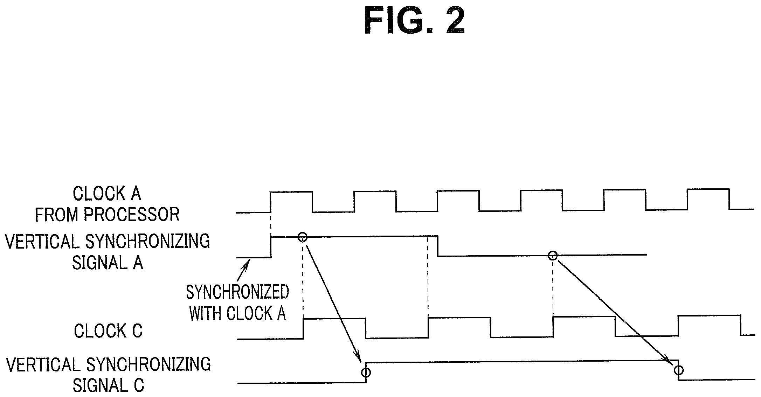

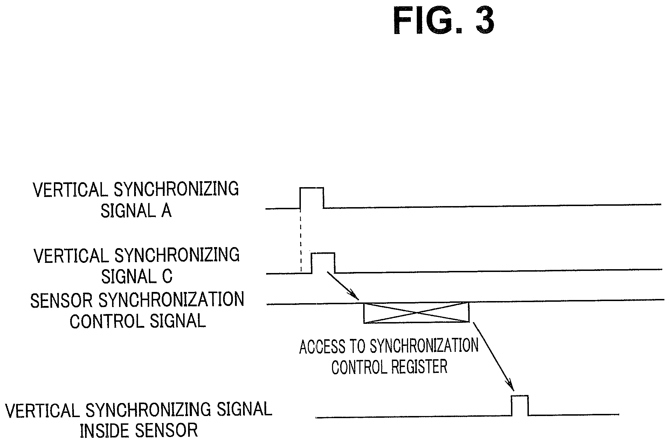

Fig. 2 is a timing chart illustrating a relation between the vertical synchronizing signal A supplied from the video processor 3 and the vertical synchronizing signal C generated on the side of the endoscope 2, in the endoscope relating to the first embodiment, and Fig. 3 is a timing chart illustrating a relation between the vertical synchronizing signal C inputted to the sensor synchronization control signal generation circuit 24 and the vertical synchronizing signal inside the CMOS sensor 21 in the endoscope relating to the first embodiment.

As illustrated in Fig. 2, the vertical synchronizing signal A from the video processor 3 received in the synchronizing signal reception circuit 25 is synchronized with the clock A generated in the processor drive clock A generation circuit 31 in the video processor 3.

On the other hand, in the present embodiment, the clock C independent of the clock A in the video processor 3 is generated in the sensor drive clock C generation circuit 23 as described above, and the synchronizing signal reception circuit 25 generates the vertical synchronizing signal C synchronized with the clock C from the vertical synchronizing signal A, based on the clock C.

Thereafter, the synchronizing signal reception circuit 25 outputs the newly generated vertical synchronizing signal C to the sensor synchronization control signal generation circuit 24.

The sensor synchronization control signal generation circuit 24 which receives the vertical synchronizing signal C transmits the sensor synchronization control signal to the register 22 which is a synchronization control register of the CMOS sensor 21 based on the vertical synchronizing signal C, as illustrated in Fig. 3.

The CMOS sensor 21 receives the sensor synchronization control signal based on the vertical synchronizing signal C in the register 22, and thereafter, generates the vertical synchronizing signal inside the sensor.

As described above, in the first embodiment, in the endoscope 2 including the CMOS sensor 21 driven by the clock C set independently of and asynchronously with the clock A in the connected video processor 3, while the clock C and the clock A are not synchronized at the clock rate, the synchronization at the frame rate is surely attained though slightly gentle between the CMOS sensor 21 and the video processor 3, and thus the endoscope that can drive the CMOS sensor 21 by the clock C of higher quality than the connected video processor 3, and does not obstruct image processing or a light source light adjustment function in the connected video processor 3 can be provided.

Note that, as described above, the center frequency of the drive clock C is set at a frequency higher than the drive clock A in the video processor 3 in the present embodiment, but regardless of the frequency, may be same as the center frequency of the clock A as long as being asynchronous with the drive clock A.

In addition, as long as being asynchronous with the drive clocks in the plurality of other video processors connectable to the endoscope 2, the center frequency of the drive clock C may be the same as the center frequency of one of the drive clocks relating to the plurality of video processors.

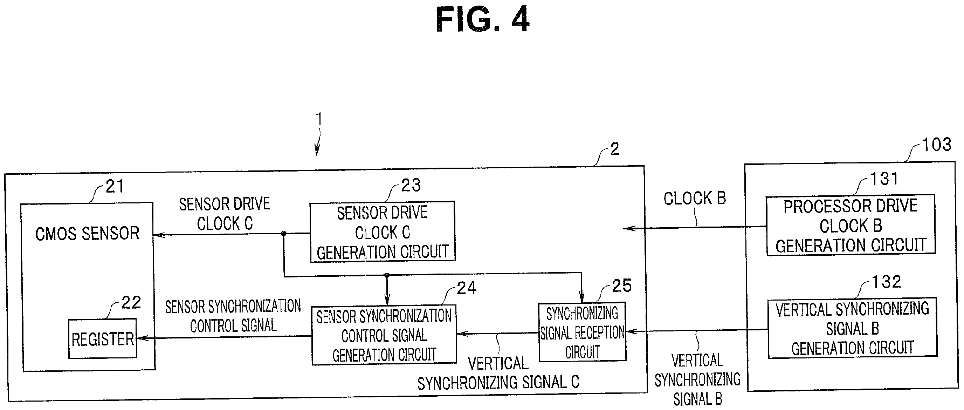

For example, as illustrated in Fig. 4, it is assumed that the endoscope 2 of the present embodiment is connected to another video processor 103 different from the video processor 3.

At the time, the video processor 103 includes a processor drive clock B generation circuit 131 configured to generate a drive clock B different from the drive clock A, and a vertical synchronizing signal B generation circuit 132 configured to generate a vertical synchronizing signal B different from the vertical synchronizing signal A.

In this case, the endoscope 2 of the present embodiment generates the drive clock C independently of and asynchronously with the clock B in the video processor 103 as described above in the sensor drive clock C generation circuit 23, and the center frequency of the drive clock C may be the same as the center frequency of the clock B as long as being asynchronous with the drive clock B.

### <Second Embodiment>

Next, a second embodiment of the present invention will be described.

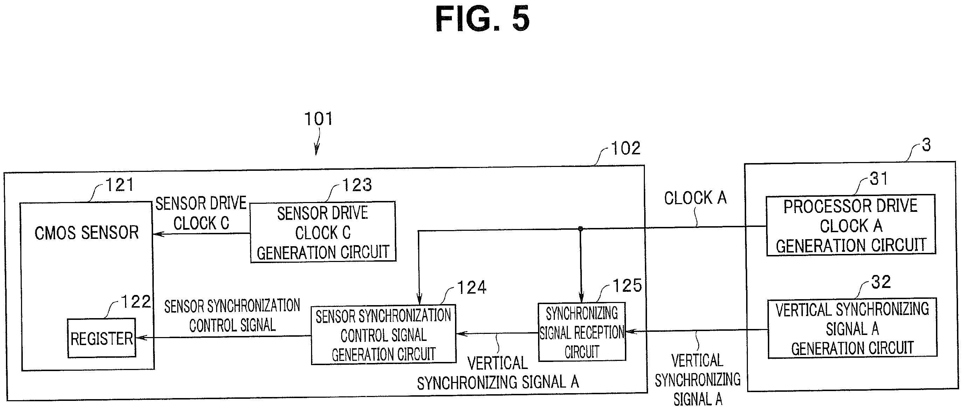

Fig. 5 is a block diagram illustrating a configuration of an endoscope system including an image pickup apparatus (endoscope) relating to the second embodiment of the present invention.

Since the configuration of the endoscope system including the image pickup apparatus of the second embodiment is basically similar to the configuration of the first embodiment, only the difference from the first embodiment will be described here and the other detailed description is omitted.

As illustrated in Fig. 5, an endoscope system 101 mainly includes an endoscope 102 configured to output the image signal of the object image similarly to the above description, the video processor 3 connectable to the endoscope 102 and capable of outputting the clock A similarly to the first embodiment, and the non-illustrated light source device similarly to the above description.

In the second embodiment as well, the video processor 3 includes the processor drive clock A generation circuit 31 configured to generate and output the predetermined drive clock A, and a vertical synchronizing signal A generation circuit 32 configured to generate and output the predetermined vertical synchronizing signal A.

The endoscope 102 includes a CMOS sensor 121 configured similarly to the first embodiment, a sensor drive clock C generation circuit 123 configured to generate a sensor drive clock (drive clock C) to be supplied to the CMOS sensor 121, a sensor synchronization control signal generation circuit 124 configured to generate the sensor synchronization control signal to be supplied to the CMOS sensor 121, and a synchronizing signal reception circuit 125 configured to receive the vertical synchronizing signal A from the video processor 3 and output the vertical synchronizing signal A to the sensor synchronization control signal generation circuit 124.

Here, in the image pickup apparatus (endoscope 2) of the first embodiment described above, not only the CMOS sensor 21 is driven by the clock C generated in a clock generation portion (sensor drive clock C generation circuit 23) inside the endoscope but also the sensor synchronization control signal generation circuit 24 and the synchronizing signal reception circuit 25 are driven by the clock C.

In addition, in the first embodiment, the synchronizing signal reception circuit 25 receives the vertical synchronizing signal A which is an external clock from the video processor 3, and newly generates and outputs the vertical synchronizing signal C based on the clock C.

Further, in the first embodiment, the sensor synchronization control signal generation circuit 24 outputs the sensor synchronization control signal to the register 22 in the CMOS sensor 21 based on the vertical synchronizing signal C.

In contrast, the sensor drive clock C generation circuit 123 in the image pickup apparatus (endoscope 102) of the second embodiment generates the drive clock C independently of and asynchronously with the clock A in the video processor 3 similarly to the first embodiment, and the CMOS sensor 121 is driven by the clock C which is the clock generated in the clock generation portion inside the endoscope.

On the other hand, in the second embodiment, the sensor synchronization control signal generation circuit 124 and the synchronizing signal reception circuit 125 are driven by the external clock, that is, the clock A generated in the processor drive clock A generation circuit 31 in the connected video processor 3 differently from the first embodiment.

In addition, in the second embodiment, the synchronizing signal reception circuit 125 receives the vertical synchronizing signal A which is the external clock from the video processor 3, and also outputs the vertical synchronizing signal A synchronized with the clock A to the sensor synchronization control signal generation circuit 124.

Further, the sensor synchronization control signal generation circuit 124 outputs the sensor synchronization control signal to a register 122 in the CMOS sensor 121 based on the inputted vertical synchronizing signal A.

Then, in the second embodiment, the CMOS sensor 121 receives the sensor synchronization control signal based on "the vertical synchronizing signal A" in the register 122, and generates the vertical synchronizing signal inside the sensor.

In this way, in the second embodiment, while the CMOS sensor 121 is driven by "the clock C" asynchronously with "the clock A" and the synchronization is not attained at the clock rate with the video processor 3, the CMOS sensor 121 generates the vertical synchronizing signal inside the sensor based on "the vertical synchronizing signal A" from the video processor 3.

### <Action of Second Embodiment>

Next, the action of the second embodiment will be described.

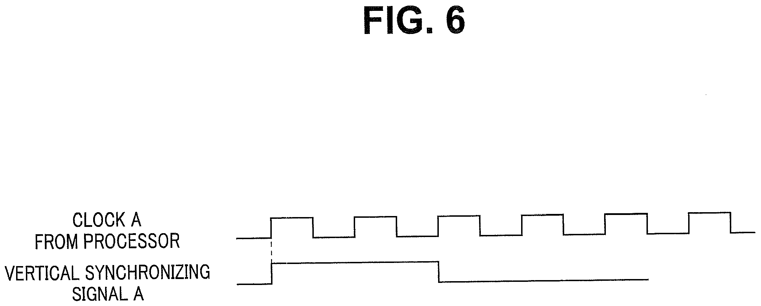

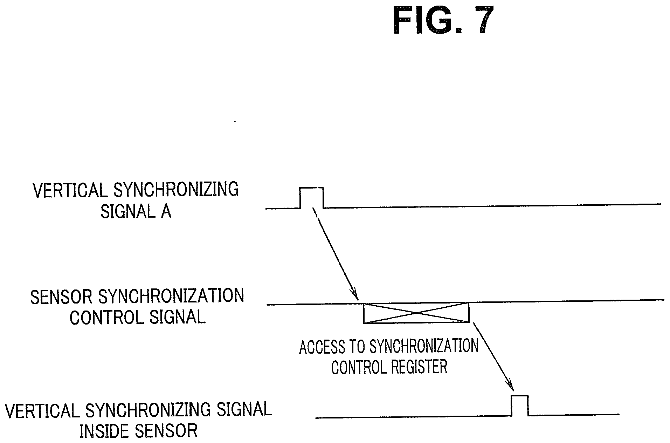

Fig. 6 is a timing chart illustrating the clock (clock A) and the vertical synchronizing signal (vertical synchronizing signal A) supplied to the sensor synchronization control signal generation circuit in the endoscope relating to the second embodiment, and Fig. 7 is a timing chart illustrating a relation between the vertical synchronizing signal inputted to the sensor synchronization control signal generation circuit and the vertical synchronizing signal inside the CMOS sensor in the endoscope relating to the second embodiment.

As illustrated in Fig. 6, while the vertical synchronizing signal A from the video processor 3 received in the synchronizing signal reception circuit 125 is synchronized with the clock A generated in the processor drive clock A generation circuit 31 in the video processor 3, in the second embodiment, the synchronizing signal reception circuit 125 is driven by the clock A from the video processor 3 as described above so that the vertical synchronizing signal to be outputted is the received vertical synchronizing signal A as it is.

In the second embodiment, the synchronization control signal generation circuit 124 transmits the sensor synchronization control signal to the register 122 which is a synchronization control register of the CMOS sensor 121 based on the vertical synchronizing signal A, as illustrated in Fig. 7.

Note that, in the present embodiment, the communication (I2C) of register control is the communication asynchronous with the sensor drive clock C, but it is possible to receive the sensor synchronization control signal based on the vertical synchronizing signal A.

That is, while the clock of high quality is demanded for the clock to be supplied to the CMOS sensor 21, demanded quality can be lower than the quality relating to the clock for communication control relating to the register control as described above so that, in the configuration of the present embodiment as well, the CMOS sensor 21 can receive the sensor synchronization control signal based on the vertical synchronizing signal A.

Then, the CMOS sensor 121 receives the sensor synchronization control signal based on the vertical synchronizing signal A in the register 122, and thereafter, generates the vertical synchronizing signal inside the sensor.

As described above, in the second embodiment as well, similarly to the first embodiment, in the endoscope 102 including the CMOS sensor 121 driven by the clock C set independently of and asynchronously with the clock A in the connected video processor 3, while the clock C and the clock A are not synchronized at the clock rate, the synchronization at the frame rate is surely attained between the CMOS sensor 121 and the video processor 3, and thus the endoscope that can drive the CMOS sensor 121 by the clock C of the higher quality than the connected video processor 3, and does not obstruct the image processing or the light source light adjustment function in the connected video processor 3 can be provided.

Note that, in the second embodiment as well, similarly to the first embodiment, the center frequency of the drive clock C is set at the frequency higher than the drive clock A in the video processor 3, but regardless of the frequency, may be same as the center frequency of the clock A as long as being asynchronous with the drive clock A.

In addition, it is similar to the first embodiment that, as long as being asynchronous with the drive clocks in the plurality of other video processors connectable to the endoscope 102, the center frequency of the drive clock C may be the same as the center frequency of one of the drive clocks relating to the plurality of video processors.

### <Third Embodiment>

Next, a third embodiment of the present invention will be described.

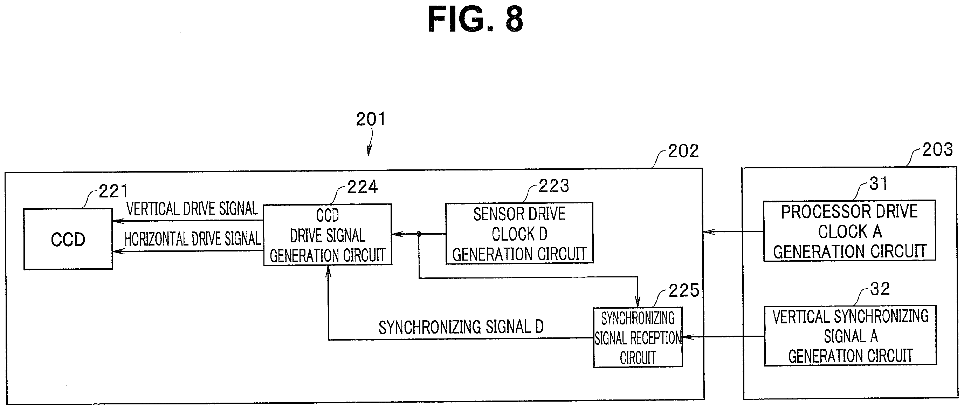

Fig. 8 is a block diagram illustrating a configuration of an endoscope system including an image pickup apparatus (endoscope) relating to an eighth embodiment of the present invention.

Both of the image pickup apparatuses (endoscopes) of the first and second embodiments described above adopt the CMOS sensor as the image pickup device, but a CCD image sensor is loaded as an image pickup device in the third embodiment.

As illustrated in Fig. 8, an endoscope system 201 relating to the third embodiment mainly includes an endoscope 202 configured to pick up an in-vivo image of an object by inserting the distal end portion into a body cavity of a subject and output an image signal of the object image, a video processor 203 configured to execute predetermined image processing on the image signal outputted from the endoscope 202 and generally control an operation of the entire endoscope system 201, and a non-illustrated light source device configured to generate illumination light to be emitted from a distal end of the endoscope 202.

The video processor 203 is, in the third embodiment as well, a video processor corresponding to the NTSC standard for example, including the processor drive clock A generation circuit 31 configured to generate and output the predetermined drive clock A, and the vertical synchronizing signal A generation circuit 32 configured to generate and output the predetermined vertical synchronizing signal A.

The endoscope 202 includes an image pickup device (CCD image sensor) 221 configured to receive the object image and photoelectrically convert the object image to an electric signal and execute predetermined image processing on the electric signal, a sensor drive clock D generation circuit 223 configured to generate a drive clock (drive clock D) to be supplied to the image pickup device 221, a CCD drive signal generation circuit 224 configured to generate a vertical drive signal and a horizontal drive signal to be supplied to the image pickup device 221, and a synchronizing signal reception circuit 225 configured to receive the vertical synchronizing signal A from the video processor 203, be driven by the drive clock C, and output a synchronizing signal D to be supplied to the CCD drive signal generation circuit 224.

For the image pickup device 221, in the present embodiment, a CCD (charge coupled device) image sensor is adopted. Hereinafter, the image pickup device 221 is also described as a CCD image sensor 221.

The sensor drive clock D generation circuit 223 generates the drive clock D independently of the clock A in the video processor 203, similarly to the first and second embodiments. The sensor drive clock D generation circuit 223 is configured by a crystal oscillator for example, and outputs to the synchronizing signal reception circuit 225 in addition to the CCD drive signal generation circuit 224.

Note that, in the present embodiment as well, the center frequency of the drive clock D is set at the frequency higher than the clock A in the video processor 203, and is set asynchronously with the clock A.

That is, in the third embodiment as well, the clock A supplied from the video processor 203 and the clock D in the sensor drive clock D generation circuit 223 are asynchronously set, that is, the configuration is such that the synchronization is not attained at the clock rate.

The synchronizing signal reception circuit 225 generates the synchronizing signal D synchronized with the clock D from the vertical synchronizing signal A based on the clock D, and outputs the synchronizing signal D to the CCD drive signal generation circuit 224.

The CCD drive signal generation circuit 224 supplies the vertical drive signals described above based on the synchronizing signal D to the CCD image sensor 221.

### <Action of Third Embodiment>

Next, the action of the third embodiment will be described.

The vertical synchronizing signal A from the video processor 203 received in the synchronizing signal reception circuit 225 is synchronized with the clock A generated in the processor drive clock A generation circuit 31 in the video processor 203.

On the other hand, in the third embodiment as well, the clock D independent of the clock A in the video processor 203 is generated in the sensor drive clock D generation circuit 223 similarly to the first embodiment, and the synchronizing signal reception circuit 225 generates a synchronizing signal D synchronized with the clock D from the vertical synchronizing signal A based on the clock D.

Thereafter, the synchronizing signal reception circuit 225 outputs the synchronizing signal D to the CCD drive signal generation circuit 224, and the CCD drive signal generation circuit 224 which receives the synchronizing signal D transmits the vertical drive signal described above to the CCD image sensor 221 based on the synchronizing signal D.

As described above, in the third embodiment as well, in the endoscope 202 including the CCD image sensor 221 driven by the clock D set independently of and asynchronously with the clock A in the connected video processor 203, while the clock D and the clock A are not synchronized at the clock rate, the synchronization at the frame rate is surely attained though slightly gentle between the CCD image sensor 221 and the video processor 203, and thus the endoscope that can drive the CCD image sensor 221 by the clock D of the higher quality than the connected video processor 203, and does not obstruct image processing or a light source light adjustment function in the connected video processor 203 can be provided.

### <Fourth Embodiment>

Next, a fourth embodiment of the present invention will be described.

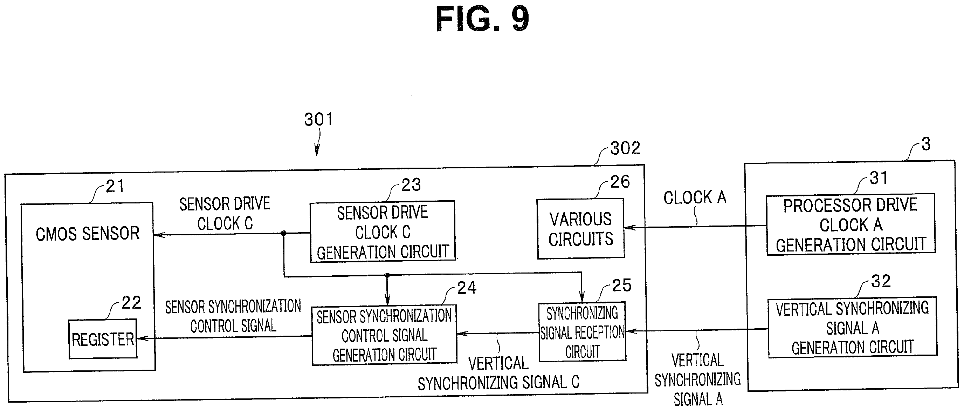

Fig. 9 is a block diagram illustrating the configuration of an endoscope system including an image pickup apparatus (endoscope) relating to the fourth embodiment of the present invention.

The image pickup apparatus (endoscope 2) of the first embodiment described above supplies the clock C generated in the clock generation portion (sensor drive clock C generation circuit 23) inside the endoscope to required circuits inside the endoscope 2 in addition to the CMOS sensor 21.

In contrast, the image pickup apparatus (endoscope 302) of the fourth embodiment includes various circuits 26 (excluding the sensor synchronization control signal generation circuit 24 and the synchronizing signal reception circuit 25) operated by the clock A in the connected video processor 3.

In this way, since the configuration of an endoscope system 301 including the image pickup apparatus of the fourth embodiment is basically similar to the first embodiment except for the point described above, the detailed description of the other configuration is omitted.

As described above, in the image pickup apparatus (endoscope) of the fourth embodiment, even in the case of partially including the circuit operated at the clock A in the connected video processor 3, the effect similar to the effect of the first embodiment described above is demonstrated.

The present invention is not limited to the embodiments described above, and can be variously changed and modified or the like without changing a subject matter of the present invention.

According to the present invention, it is possible to provide an image pickup apparatus capable of not causing troubles in image processing or light adjustment of a light source and coping with various video processors of different clock frequencies, in the image pickup apparatus including an image pickup device driven by a clock set independently of and asynchronously with a clock in a connected video processor.

The present application is filed with Japanese Patent Application No. 2015-124907 filed in Japan on June 22, 2015 as a base of a claim of priority, and the above-described disclosure content is incorporated in the present description and claims by reference.

## Claims

1. An image pickup apparatus connectable to a processor including a first clock generation portion configured to generate a predetermined first clock and a first vertical synchronizing signal generation portion configured to generate a predetermined first vertical synchronizing signal, the image pickup apparatus comprising:
an image pickup device configured to pick up an image of an object;
a second clock generation portion configured to generate a second clock which is a clock to drive the image pickup device and is set independently of and asynchronously with the first clock;
a second vertical synchronizing signal generation portion configured to receive the first vertical synchronizing signal and generate a second vertical synchronizing signal including a period synchronized at least at a frame rate with the first vertical synchronizing signal; and
a sensor synchronization control signal generation portion configured to generate a sensor synchronization control signal which controls the image pickup device according to the second vertical synchronizing signal.

2. The image pickup apparatus according to claim 1,
wherein the second vertical synchronizing signal generation portion generates the second vertical synchronizing signal according to the second clock.

3. The image pickup apparatus according to claim 2,
wherein the sensor synchronization control signal generation portion generates the sensor synchronization control signal according to the second clock.

4. The image pickup apparatus according to claim 1,
wherein the second vertical synchronizing signal generation portion generates the second vertical synchronizing signal according to the first clock.

5. The image pickup apparatus according to claim 4,
wherein the sensor synchronization control signal generation portion generates the sensor synchronization control signal according to the first clock.
